**Europäisches Patentamt**

(19) **European Patent Office**

**Office européen des brevets**

(11) Veröffentlichungsnummer: **0 087 728**
**B1**

(12) **EUROPÄISCHE PATENTSCHRIFT**

(45) Veröffentlichungstag der Patentschrift:
**23.10.85**

(51) Int. Cl.⁴: **G 01 N 33/546**

(21) Anmeldenummer: **83101692.8**

(22) Anmeldetag: **22.02.83**

(54) **Immunologisches Latex-Agglutinationsverfahren.**

(30) Priorität: **25.02.82 DE 3206729**

(43) Veröffentlichungstag der Anmeldung:
**07.09.83 Patentblatt 83/36**

(45) Bekanntmachung des Hinweises auf die Patenterteilung:
**23.10.85 Patentblatt 85/43**

(84) Benannte Vertragsstaaten:
**AT BE CH DE FR GB IT LI LU NL SE**

(56) Entgegenhaltungen:
**Keine**

(73) Patentinhaber: **BEHRINGWERKE Aktiengesellschaft,
Postfach 1140, D-3550 Marburg 1 (DE)**

(72) Erfinder: **Toth, Tibor, Dr., Gründeberg 1,
D-3550 Marburg/Lahn (DE)**
Erfinder: **Münscher, Gerhard, Dr., Alte Hute 9,
D-3550 Marburg/Lahn (DE)**

(74) Vertreter: **Meyer-Dulheuer, Karl-Hermann, Dr. et al,
HOECHST Aktiengesellschaft Zentrale Patentabteilung
Postfach 80 03 20, D-6230 Frankfurt/Main 80 (DE)**

**0 087 728**

**Beschreibung**

Die Erfindung betrifft ein Latex-Agglutinationsverfahren zum Nachweis oder zur Bestimmung eines der Partner einer Antigen-Antikörper-Reaktion in Gegenwart eines Gammaglobulins, das keine für einen der Partner spezifischen Antikörper enthält.

Bekanntlich verbinden sich spezifische Antikörper mit den entsprechenden Antigenen oder Haptenen. Von dieser Reaktion wird in vielen Immunoassay-Verfahren Gebrauch gemacht. Menschliche Seren können auf die Anwesenheit eines bestimmten Antigens unter Verwendung des entsprechenden Antikörpers beispielsweise mittels der kompetitiven Bindungsreaktion oder der Latex-Agglutinationsreaktion untersucht werden. Diese und ähnliche Verfahren sind dem Fachmann wohl bekannt.

Eine Schwierigkeit, die in derartigen Methoden auftreten kann, besteht darin, daß andere in dem zu untersuchenden Serum vorhandene Bestandteile stören können. Insbesondere enthält das menschliche Serum das Protein C1q (eine Komplementkomponente) und Rheumafaktoren (RF). Diese beiden Substanzen binden sich gleichfalls an den Antikörper. Außerdem können die Mengen an Rheumafaktor und C1q in menschlichen Seren in sehr weiten Grenzen variieren, weshalb es normalerweise erforderlich ist, die Seren vorher einer Behandlung zur Inaktivierung von C1q oder Entfernung endogener Rheumafaktoren zu unterziehen. Andernfalls können die Ergebnisse und insbesondere die quantitativen Bestimmungen beträchtliche Fehler aufweisen.

Der Nachweis von Antigenen oder Antikörpern mit Hilfe der Latex-Agglutinationsreaktion hat den Vorteil, daß die Durchführung einfach ist und die Testergebnisse in sehr kurzer Zeit erhalten werden.

Es wurde nun überraschenderweise gefunden, daß die vorstehend genannten Schwierigkeiten, verursacht durch eine nicht-spezifische Agglutination von Latex-Teilchen, verhindert werden können, indem die Latex-Agglutinationsreaktion in Gegenwart eines Gammaglobulins oder einer Gammaglobulinfraktion durchgeführt wird, das beziehungsweise die weder mit dem zu bestimmenden Antigen oder Antikörper noch mit dem an die Latexpartikel gebundenen Partner reagiert.

Gegenstand der Erfindung ist demnach ein Latex-Agglutinationsverfahren, dadurch gekennzeichnet, daß man mit einem Partner einer immunologischen Reaktion beladene Latex-Teilchen mit einer Lösung des entsprechenden Partners in Gegenwart eines Gammaglobulins zusammenbringt, das keine für einen der Partner spezifischen Antikörper enthält. Als solche Gammaglobuline zur Verhinderung einer unspezifischen Agglutination eignen sich tierische Gammaglobuline oder hitzeaggregierte humane Gammaglobuline. Gammaglobulin-Fraktionen werden mittels bekannter Verfahren, beispielsweise Ammonsulfat-Fällung oder Ionenaustauscher-Chromatographie, gewonnen.

Das erfindungsgemäße Verfahren ist auf alle Reaktionen zum Nachweis von immunologisch aktiven Substanzen, die im Blut (Serum oder Plasma) von Säugetieren, insbesondere vom Menschen, enthalten sind, anwendbar. Beispiele solcher immunologisch aktiver Substanzen sind Serumproteine.

Als Latex-Teilchen, die mit den immunologisch aktiven Substanzen beladen (sensibilisiert) werden, kommen alle Latices in Frage, die für den Latex-Agglutinationstest geeignet sind. Als Beispiele seien Homo- und Copolymere von Styrol genannt. Bevorzugt werden Latices mit einer Teilchengröße von 0,05 bis 0,6 μm.

Die Sensibilisierung der vorstehend genannten Latex-Teilchen mit den Antigenen oder Antikörpern kann nach einer bekannten Methode durchgeführt werden. Bevorzugt wird das Latex mit Antikörpern gegen Serumproteine wie Myoglobin, Beta 2-Mikroglobulin oder Immunglobulin E, humane Hormone wie Human-Choriogonadotropin, Enzyme wie Pankreas-Lipase oder tierische Hormone wie Pregnant mare serum gonadotropin beladen. Es kann dann wie folgt sensibilisiert werden: Aus einem Antiserum werden die Gammaglobuline in üblicher Weise, beispielsweise mit Ammoniumsulfat, ausgefällt, die Gammaglobulinfraktion dialysiert und auf 30—50 g/l konzentriert. Es können auch reine Antikörperlösungen immunadsorptiv gewonnen und auf 2—10 g/l konzentriert werden. Eine Suspension der Latex-Teilchen mit einer Konzentration von etwa 100 g/l wird mit der Antikörper-Lösung versetzt und 0,5—5 Stunden bei einer Temperatur zwischen 20 und 60° C inkubiert. Der nicht an Latex-Teilchen gebundene Anteil der Antikörper kann durch Zentrifugation und Resuspension des Feststoffes entfernt werden. Zum Gebrauch kann das Reagenz in einer Pufferlösung, vorzugsweise Glycin-NaCl-Puffer von pH 7—8,5, resuspendiert werden, die gegebenenfalls mit einem Protein, beispielsweise mit Rinder- oder Human-Albumin, versetzt werden kann. Die zur Herstellung des Gammaglobulins verwendbaren Antiseren werden durch immunisieren von Tieren, besonders Kaninchen, Schafe, Ziegen, mit einem Protein humanen oder tierischen Ursprungs, die das im Test zu bestimmende Protein nicht enthalten dürfen, hergestellt.

Beispiele sind: Anti-Human-IgG-Serum vom Kaninchen, Anti-Human-IgM-Serum vom Kaninchen, Anti-Schaf-Erythrozyten-Serum vom Kaninchen, Anti-Human-IgG-Serum vom Schaf, Anti-Kaninchen-γ-Globulin-Serum vom Schaf. Besonders geeignet ist das Anti-Schaf-Erythrozyten-Serum vom Kaninchen. Die Immunisierung wurde nach bekannten Methoden durchgeführt. Immunisierungsdosis und -zeit ergeben sich aus der Immunogenität und dem Molekulargewicht des Proteins.

Die verwendeten Antikörper-Lösungen enthalten keine Antikörper gegen das an die Latex-Partikel gebundene tierische Antiserum, das heißt, sie sind gewöhnlich von derselben Tierspezies.

Die Gammaglobulin-Lösung wird der Lösung, in der ein Antigen oder ein Antikörper bestimmt oder nachgewiesen werden soll, bevorzugt im Verhältnis 1 : 0,1 bis 2 (v : v) zugesetzt. Folgende Beispiele

2

# 0 087 728

erläutern das erfindungsgemäße Verfahren.

## Beispiel 1

Nach dem Stand der Technik hergestelltes Latex-Reagenz, welches als spezifische Antikörper immunadsorptiv gewonnene Antikörper gegen Human-Myoglobin vom Kaninchen an Latex-Teilchen gebunden enthält, wurde erfindungsgemäß in folgendem Test eingesetzt. Die Empfindlichkeit des Reagenzes wurde an einem Standard auf etwa 80 ng/ml eingestellt und dieser Test wie folgt ausgeführt:

1 Tropfen verdünntes zu prüfendes Human-Serum (50 µl) wurde auf ein Feld einer Testplatte gegeben, 5 µl Gammaglobulinlösung, die keine Antikörper gegen Human-Myoglobin enthält, nämlich Anti-Schaf-Erythrozyten-Serum vom Kaninchen, zugegeben und 1 Tropfen (25 µl) Latex-Myoglobin-Reagenz zugesetzt. Nach Durchmischen mittels eines Rührstäbchens wurde die Testplatte rotierend bewegt und nach drei Minuten auf Agglutination geprüft. Bei negativem Ergebnis wurde die Testplatte weiter rotierend bewegt und innerhalb von 2 Minuten erneut auf Agglutination geprüft.

Die folgende Tabelle veranschaulicht die Zuverlässigkeit des Verfahrens bei der Bestimmung von Myoglobin im Serum:

| Serum Nr. | Anwesenheit von Rheumafaktoren | Myoglobin RIA (ng/ml) | Latex-Test gemäß Stand der Technik | Erfindung |
|---|---|---|---|---|
| 1 | 0 | 110 | + | + |
| 2 | 0 | 320*) | + | + |
| 3 | 0 | 75*) | − | − |
| 4 | 0 | 20 | − | − |
| 5 | + | 40 | + | − |
| 6 | + | 20 | + | − |
| 7 | + | 10 | + | − |
| 8 | + | 120 | + | + |

*) Die klinisch interessierende Grenze liegt bei etwa 80 ng/ml.

Wie die Tabelle zeigt, können Rheumafaktoren im Myoglobin-Latex-Test falsch positive Ergebnisse bewirken. In den Seren 5, 6 und 7 fiel der Latex-Test nach dem Stand der Technik positiv aus (4. Spalte), obwohl der Myoglobin-RIA (Radio Immuno Assay) als Standardtest negativ war, weil er 40, 20 beziehungsweise 10 ng/ml Myoglobin anzeigte, welche Werte unter dem klinisch vorgegebenen Wert von etwa 80 ng/ml liegen. Der erfindungsgemäße Test (letzte Spalte) ergibt dagegen die gewünschte Übereinstimmung mit dem Radio-Immuntest.

In den Seren 1 bis 4, die keine störenden Bestandteile (Rheumafaktoren) enthalten, werden mit beiden Latex-Testen gleiche und mit RIA übereinstimmende Ergebnisse erhalten.

## Beispiel 2

Nach dem Stand der Technik hergestelltes Latexreagenz, welches als spezifische Antikörper Antikörper gegen PMSG von Kaninchen an Latex-Teilchen gebunden enthält, wurde erfindungsgemäß in folgendem Test eingesetzt. Die Empfindlichkeit des Reagenzes wurde mit Hilfe eines Standards auf etwa 2 IU/ml eingestellt und dieser Test wie folgt ausgeführt:

1 Tropfen unverdünntes zu prüfendes Stuten-Serum (50 µl) wurde auf ein Feld einer Testplatte gegeben, 25 µl Gammaglobulinlösung (Anti-Schaf-Erythrozyten-Serum vom Kaninchen) zugegeben und 1 Tropfen (25 µl) Latex-Pregnant mare serum gonadotropin-Reagenz zugesetzt. Nach guter Durchmischung mittels eines Rührstäbchens wurde die Testplatte rotierend bewegt und nach einer Minute auf Agglutination geprüft. Bei negativem Ergebnis wurde die Testplatte weiter rotierend bewegt und innerhalb von 2 Minuten erneut auf Agglutination geprüft.

3

**0 087 728**

Die folgende Tabelle zeigt die vergleichenden Ergebnisse von Trächtigkeitstests an Stuten:

| Serum von Stute | Latex-Test gemäß Erfindung | Stand der Technik | Rektal-Untersuchung |
|---|---|---|---|
| 1 | — | — | — |
| 2 | — | + | — |
| 3 | — | + | — |
| 4 | + | + | + |
| 5 | + | + | + |
| 6 | + | + | + |

Zwei der Seren reagierten im Latextest nach dem Stand der Technik positiv (Seren 2 und 3), obwohl die Rektaluntersuchung ergab, daß die Stuten nicht trächtig waren. Die Ergebnisse des erfindungsgemäßen Tests stimmten dagegen in allen Fällen mit dem Resultat der Rektaluntersuchung überein.

**Patentansprüche**

1. Latex-Agglutinationsverfahren zum Nachweis oder zur Bestimmung eines Partners einer immunologischen Reaktion mittels des entsprechenden Partners, dadurch gekennzeichnet, daß man den Nachweis oder die Bestimmung in Gegenwart eines Gammaglobulins vornimmt, das keine Antikörper enthält, die für einen der Partner spezifisch sind.

2. Verfahren nach Anspruch 1, dadurch gekennzeichnet, daß das Gammaglobulin eine wäßrige Lösung einer tierischen Gammaglobulinfraktion ist.

3. Verfahren nach Anspruch 1, dadurch gekennzeichnet, daß das Gammaglobulin ein Anti-Schaf-Erythrozyten-Serum von einem Säuger ist.

4. Verfahren nach Anspruch 1, dadurch gekennzeichnet, daß das Gammaglobulin ein Anti-Schaf-Erythrozyten-Serum vom Kaninchen ist.

**Claims**

1. A latex agglutination process for the detection or determination of a partner of an immunologic reaction by means of the corresponding partner, which comprises carrying out the detection or determination in the presense of a gamma globulin containing no antibodies specific for either of the partners.

2. The process of claim 1, wherein the gamma globulin is an aqueous solution of an animal gamma globulin fraction.

3. The process of claim 1, wherein the gamma globulin is an anti-sheep-erythrocyte-serum from a mammal.

4. The process of claim 1, wherein the gamma globulin is an anti-sheep-erythrocyte-serum from the rabbit.

**Revendications**

1. Procédé d'agglutination sur latex pour la détection ou la détermination d'un partenaire d'une réaction immunologique, au moyen du partenaire correspondant, caractérisé en ce qu'on effectue la détection ou la détermination en présence d'une gammaglobuline qui ne contient pas d'anticorps spécifiques pour l'un des partenaires.

2. Procédé selon la revendication 1, caractérisé en ce que la gammaglobuline est une solution aqueuse d'une fraction de gammaglobuline animale.

3. Procédé selon la revendication 1, caractérisé en ce que la gammaglobuline est un sérum de mammifères anti-érythrocytes de mouton.

4. Procédé selon la revendication 1, caractérisé en ce que la gammaglobuline est un sérum de lapin anti-érythrocytes de mouton.

4